# EUROPEAN PATENT SPECIFICATION

(11) **EP 2 398 457 B1**
(45) Date of publication and mention of the grant of the patent: **10.08.2016**
(21) Application number: 10708746.2
(22) Date of filing: 23.02.2010
(51) Int. Cl.: A61K 9/12, A61K 47/10, A61K 47/44, A61K 31/573

(54) **A TOPICAL FORMULATION OF LOW LEVEL CLOBETASOL PROPIONATE FOR TREATING DISORDERS OF THE SKIN AND MUCOUS MEMBRANES**
TOPISCHE FORMULIERUNG VON NIEDRIGDOSIERTEM CLOBETASOLPROPIONAT ZUR BEHANDLUNG VON HAUT- UND SCHLEIMHAUTERKRANKUNGEN
FORMULATION TOPIQUE FAIBLEMENT DOSÉE EN PROPIONATE DE CLOBÉTASOL POUR TRAITER DES AFFECTIONS DE LA PEAU ET DES MUQUEUSES

(30) Priority: 23.02.2009 US 208369 P; 22.02.2010 US 660181
(43) Date of publication of application: 28.12.2011
(73) Proprietor: Abigo Medical Ab, 436 33 Askim (SE)
(72) Inventor: SMITH, Jan G., S-436 39 Askim (SE)
(74) Representative: Wynne-Jones, Laine and James LLP
(86) International application number: PCT/EP2010/052243
(87) International publication number: WO 2010/094806

(56) References cited:
- EP-A1- 2 016 935
- WO-A1-2007/104895
- FR-A1- 2 912 655

## Description

### BACKGROUND OF THE INVENTION

### Field of the Invention

The present invention relates to new topical formulations with a high chemical stability of a low dose clobetasol propionate, and methods of use, suitable for the topical treatment of skin and mucous membrane conditions associated with disorders including psoriasis, eczema, and other forms of dermatitis and also topical use in and associated with the mouth, such as lichen planus. In particular, the invention relates to an aqueous vehicle based on propylene glycol as a solvent and moisture-retaining agent, and macrogol-glycerol hydroxystearate as a non-ionic emulsifier being capable of holding surprisingly low concentrations of clobetasol. More specifically the invention relates to a vehicle holding concentrations about 0.005% to about 0.05% by weight of 17-clobetasol propionate, more preferably about 0.02 to 0.025 %, even more preferably 0.025% by weight of 17-clobetasol propionate.

### Background of the invention

Dermatitis is a superficial inflammation of the skin, characterized by vesicle formation, erythema, edema, oozing, scaling or crusting lesions, and intense itching. Different types of dermatitis can be distinguished: contact dermatitis, caused by irritants in contact with the skin or by non-irritating substances, to which the subject is allergic; atopic dermatitis, characterized by strong itching and a chronic course; and seborrheic dermatitis, a scaling disease mainly affecting the face and scalp.

One particular form of dermatitis is psoriasis, a chronic, inflammatory, hyperproliferative recurring disease, which affects the skin and joints. In its most typical form, it causes thick, red, scaly patches, called psoriatic plaques, to appear on the skin. Skin rapidly accumulates at these sites and takes on a silvery-white appearance. Plaques most often occur on the skin of the elbows, knees, scalp, lower back, face, palms, and soles of the feet but can affect any skin site.

The severity and course of psoriasis can vary greatly depending on the individual, but in general this condition recurs throughout the life of the individual causing significant psychological and social distress, and significantly impacting on quality of life. About 15 percent of people with psoriasis have joint inflammation that produces arthritis symptoms. This condition is called psoriatic arthritis. The exact mechanism which triggers the abnormal cell proliferation is not known, though researchers believe psoriasis occurs when faulty signals in the immune system cause skin cells to grow too rapidly. There are many treatments available but because of its chronic recurrent nature psoriasis is a challenge to treat.

Another form of dermatitis or inflammation of the epidermis is eczema, persistent skin conditions including dryness and recurring skin rashes. Other skin and mucous membrane conditions include disorders in the mouth, in the vagina, anus, ear and the eyes. Lichen planus is a chronic mucocutaneous disease that affects the skin and the oral mucosa, and presents itself in the form of papules, lesions or rashes.

Topical treatments performed by applying agents to the skin that slow down or normalize the excessive cell reproduction and reduce inflammation are usually the first line of defense in treating psoriasis and other skin disorders. Topical corticosteroids are the most prescribed treatment for mild to moderate psoriasis and may also complement other psoriasis treatments for moderate to severe psoriasis. This is true for many of the other skin and mucus associated conditions above. Their efficacy is dependent on the ability of the corticosteroid molecule to activate corticosteroid receptors and the ability of the vehicle to deliver the active drug through the skin. The active ingredient is commonly formulated in a moisturizing base in several forms, including lotions and ointments, which provide a layer of oil on the surface of the skin, helping to prevent water from evaporating from the skin surface. This helps reduce the dryness, scaling and itching of skin conditions such as eczema.

If corticosteroids are used long-term on large areas of skin or mucous membranes, they can be absorbed into the body. This increases the risk of local side effects such as skin thinning, and of systemic side effects, such as adrenal suppression, where the adrenal glands become unable to regulate hormones being released in the body; and tachyphylaxis, where the body develops immunity to a certain treatment regimen. Moreover, the need for long-term application may be a daunting task for many patients. Poor adherence to treatment over time, due to an undesirable vehicle, may result in poor treatment outcomes.

Unsatisfactory treatment of the disorder has a considerable adverse impact on the patient's quality of life with patients complaining about the messiness of the topical agents used. Therefore, it is essential to have a formulation delivering topical steroids properly to avoid unnecessary side effects and ensure a positive treatment outcome.

Clobetasol propionate is a very potent corticosteroid and is one of the most common topical therapies used for psoriasis. The efficiency, tolerability, and applicability of topical agents are directly related to employed vehicles. Thus to achieve optimum topical therapy, vehicle composition, and its physical action on the skin is important. Common vehicles are complex mixtures consisting of diverse ingredients belonging to various groups, i.e. hydrophilic and lipophilic bases, emulsifiers, gel-forming agents, preservatives, and antioxidants. The proper choice of the vehicle, with respect to the properties of the incorporated active ingredient, is of paramount importance to maximize the therapeutic effect at the site of application.

Clobetasol propionate is commercially available in compositions for topical application in form of lotion, spray, cream or shampoo, in a weight concentration of 0.05%. Moreover, various topical pharmaceutical compositions comprising clobetasol propionate has been proposed in the prior art, claiming the use of particular carriers or excipients.

US Patent No. 5,972,920 claims a formulation characterized by a carrier compound formed of a combination of two components in a volume ratio of about 50/50, wherein a first carrier component is selected from the group consisting essentially of ethyl alcohol and isopropyl alcohol and a second carrier component is selected from the group consisting essentially of isopropyl myristate, isopropyl palmitate, octyl palmitate, octyl isononanoate, and isocetyl stearate. The formulation also comprises an anionic surfactant.

WO 2006115987 in the name of Dow Pharmaceutical Sciences provides a method for treating psoriasis by spraying a pharmaceutical composition containing an effective amount of clobetasol propionate onto the skin with psoriasis, using a daily treatment for at least 4 weeks. The preferred composition is a spray formulation of clobetasol propionate 0.05%, containing alcohol, isopropyl myristate, an anionic surfactant such as sodium lauryl sulfate, and optionally an antimicrobial compound such as an antifungal compound like undecylenic acid.

US Patent No. 6,579,512 refers to a pharmaceutical topical spray composition of a corticosteroid, consisting essentially of clobetasol propionate and isopropyl myristate. The composition also comprises an alcohol and a propellant.

According to US Patent No. 5,990,100 isopropyl myristate is an active agent for treating psoriasis and can be combined as the first active agent with a known anti-psoriatic agent (second active agent) giving a more effective multi-active-agent composition. The known anti-psoriatic agent can be a corticosteroid. The composition also comprises sodium lauryl sulphate and Polysorbate 80 (polyoxyethylene (20) sorbitan monooleate) as emulsifiers (or dispersants or surfactants) in a water/ethanol vehicle. In a less preferred embodiment the vehicle may be an oil system, such as fat or oil or synthetic fat such as petrolatum.

WO 2004093722 reports a composition comprising at least one polyhydroxy lactone or polyhydroxy acid compound selected from a polyhydroxy-aldonic acid, a polyhydroxy-aldonic lactone, a polyhydroxy-alduronic acid, a polyhydroxy-alduronic lactone, a polyhydroxy-aldaric acid, polyhydroxy-aldaric lactone, and an organic acid lactone having two or more hydroxyl or ketohydroxyl groups. Active agents, including clobetasol propionate, may be incorporated into the composition.

Galderma disclosed oil-in-water (O/W) anti-inflammatory emulsions, containing: a) a therapeutically effective amount of at least one steroidal anti-inflammatory agent, notably clobetasol propionate; b) a pro-penetrating system which includes at least one pro-penetrating glycol and at least one other pro-penetrating agent; a polymeric or non-polymeric emulsifier or one gelling agent (WO2007104895 and WO2007104897). In both cases preferred pro-penetration system are: propylene glycol and isosorbide dimethyl; propylene glycol and ethanol; propylene glycol, monoethylether of diethylen glycol and propylene glycol laurate; propylene glycol and methyl pyrrolidone; propylene glycol, isosorbide dimethyl and ethanol; propylene glycol, methyl pyrrolidone and oleic alcohol.

In one preferred embodiment, the composition comprises from 0.025% to 0.5% by weight and preferentially 0.05% by weight of clobetasol propionate relative to the total weight of the composition. The pro-penetrating agent is preferably selected from propylene glycol, dipropylene glycol and propylene glycol dipelargonate. More preferably, the pro-penetrating agent is propylene glycol.

WO2007020349 claims a topically applicable oil-in-water emulsion containing at least one biologically active agent and also comprising: a) a fatty phase, the amount thereof ranging from about 35% to 50% by weight; b) from about 1% to 15% by weight of a nonionic emulsifying system; c) from about 1% to 30% by weight of at least one pro-penetrating agent; and d) from about 5% to 50% by weight of water. The fatty phase is emulsified by means of a nonionic emulsifying system (nonionic surfactant) with a predominant hydrophilic fraction.

EP1360958 relates to a liposomic formulation of clobetasol propionate. This kind of composition allows the use of low concentrations of the active ingredient (between 0.01 and 0.03%), rendering high concentration of the product in the skin. Preferred concentrations range from 0.02 to 0.025% of clobetasol propionate, the optimum being 0.025%.

Other documents of the prior art propose the use of clobetasol in combination with at least a further active ingredient, such as calcitriol (Vitamin D) (see for example WO 2008110815, EP1854466, EP 1875916, US Patent No. 2005281850, US Patent No. 2006009426 and FR 2848454); a progesterone derivative (EP1473300); a prostaglandin (WO03092617); or tazarotene (CA2282682). FR 2912655 discloses a two-compartment product made up of a first compartment having an anhydrous composition comprising an analogue of vitamin D, and a second compartment having a composition comprising a corticosteroid, preferably propionate clobetasol.

EP 2016935 discloses a pharmaceutical composition for topical application of poorly soluble compounds.

As shown above, dermatological corticosteroids and in particular clobetasol propionate have been provided in a variety of topical formulations such as creams, lotions, gels and the like in attempts to increase the delivery efficiency. However, while enabling direct, localized application of the active agent to the skin surface, these formulations have not provided a complete solution to the problems connected with the topical application of corticosteroids.

A first problem in the preparation of dermatological compositions is delivery efficiency. A second problem is stability, both of the active ingredient and of the auxiliary components in the formulation. A further problem is tolerability, in particular with respect to excipients that would not cause irritation.

Moreover, topical corticosteroids can be absorbed through the skin and travel into the bloodstream. This may constitute a problem, in particular when widespread areas of body is treated, and can provoke unwanted side effects, like hypothalamic pituitary adrenal (HPA) axis suppression. Thus treatment with a low dose of clobetasol would be desired. The earlier known formulations with low dose, for example manufactured extempore by Apoteksbolaget AB in Sweden, are stable for a maximum of 3 months.

Ointments are commonly prescribed for psoriasis, in part because of the perception that they are more potent and in part because of the perception that moisturizing psoriasis plaques is inherently beneficial. Nevertheless, ointment-based vehicles are among the least appealing to patients due to their messiness and greasy feel. Bothersome aspects of the vehicle likely reduce patients' adherence to treatment. Discontinuation of topical steroids should be obviated, to avoid a psoriasis "rebound".

Topical clobetasol propionate is currently one of the most used treatments for psoriasis and its safety and efficacy is well defined in the medical literature. However, current formulations of clobetasol present disadvantages. Cream and ointment are greasy and difficult to apply on large areas, which disadvantages negatively impact treatment compliance and quality of life. The use of other pharmaceutical forms is restricted to short periods of time due to the risk of side effects.

A liposomal formulation has been also proposed in the patent literature, with a reduced amount of the active ingredient. However, liposomes are difficult to prepare, expensive and present problem of chemical stability. Thus an efficient non-liposomal formulation, with high chemical stability and a capability to contain low doses of clobetasol, for the successful treatment of psoriasis is desirable.

The present invention showed to our surprise that an aqueous vehicle based on propylene glycol and macrogol-glycerol hydroxystearate is capable of holding surprisingly low concentrations of clobetasol and at the same time the formulation showed to have a very good chemical stability, consequently providing the product a surprisingly long durability.

### SUMMARY OF THE INVENTION

The present invention to provide improved pharmaceutical formulations containing clobetasol propionate or the like for use in the treatment of psoriasis and other inflammatory skin and mucous membrane disorders including psoriasis, eczema, atopic dermatitis, contact dermatitis and seborrhoeic dermatitis and other forms of dermatitis and also topical use associated with the mouth, such as lichen planus. The formulation has a good chemical stability, resulting in a long durability. Further disclosed is an improved method for the treatment of psoriasis and related inflammatory skin disorders using the novel formulations based on propylene glycol as a solvent and moisture-retaining agent, and macrogol-glycerol hydroxystearate as a non-ionic emulsifier.

In this respect, before explaining at least one embodiment of the invention in detail, it is to be understood that the invention is not limited in its application to the details of construction and to the arrangements of the components set forth in the following description . The invention is capable of other embodiments and of being practiced and carried out in various ways. Also, it is to be understood that the phraseology and terminology employed herein are for the purpose of the description and should not be regarded as limiting.

### DETAILED DESCRIPTION OF THE INVENTION AND PREFERRED EMBODIMENTS THEREOF

The present invention provides improved pharmaceutical formulations containing clobetasol propionate for use in the treatment of psoriasis and other inflammatory skin and mucous membrane disorders including psoriasis, eczema, atopic dermatitis, contact dermatitis and seborrhoeic dermatitis and other forms of dermatitis and also topical use associated with the mouth, such as lichen planus. The formulation has a good chemical stability, resulting in a long durability.

In particular the invention relates to an aqueous vehicle based on propylene glycol as a solvent and moisture-retaining agent, and macrogol-glycerol hydroxystearate as a non-ionic emulsifier being capable of holding surprisingly low concentrations of clobetasol. More specifically the invention relates to a vehicle holding concentrations about 0.005% to about 0.05 % by weight of 17-clobetasol propionate, more preferably about 0.02 to 0.025 %, even more preferably 0.025% by weight of 17-clobetasol propionate.

The pharmaceutical compositions of the present invention are preferably provided in the form of a lotion, a gel, oromucosal gel, a solution, a liquid suspension or dispersion, or a spray comprising clobetasol propionate in a suitable pharmaceutically acceptable medium. In certain embodiments, the composition can be in the form of an oil, emulsion or a semisolid preparation like a cream or ointment, in combination with a suitable base for this kind of compositions.

A preferred form is a spray, because it is easy to use and not as messy as other topical medications. For spray administration the composition may be packaged in a bottle fitted with a spray pump closure that can be mechanically actuated by a patient, or in an aerosol can or bottle fitted with an actuator and charged with a propellant.

As said before, clobetasol propionate has been proposed in the prior art in several pharmaceutical compositions for topical application. In said formulations, the clobetasol propionate is present in about 0.01 to 10% (%w/w), more preferably in about 0.01% to 1% (%w/w), most preferably in the amount of 0.05%w/w relative to the total weight of the composition.

Only in the case of a liposomal formulation, wherein the active ingredient has to be encapsulated in liposomes, has it been possible to reduce its concentration.

Applicants have now found that a highly effective topical skin preparation for the treatment of psoriasis, and other disorders of the skin and mucous membranes, can be provided by utilizing as a vehicle a combination of water and glycol as a solvent to favor the skin penetration and retention of moisture, together with a particular nonionic emulsifying compound (also referred to as dispersant or surfactant) to ensure complete dispersion of the active ingredient in the vehicle and increase the physical stability of the composition.

A preferred example of a suitable glycol for delivering clobetasol propionate to the epidermis is propylene glycol or 1,2-propanediol. The preferred emulsifier is macrogol-glycerol hydroxystearate 40 or Cremophor® RH 40.

According to a preferred embodiment of the present invention, the vehicle contains propylene glycol as a dissolvent at a proportion between 4% and 10%, in addition to water at a proportion between 70% and 85% and to macrogol-glycerol hydroxystearate as emulsifier at a proportion between 4% and 10%. Most preferably, the propylene glycol is present in the amount of about 5.000 % (%w/w), water in the amount of about 80% and macrogol-glycerol hydroxystearate in the amount of about 5.000 % (%w/w). On a weight basis, the ratio of solvent compound to emulsifier compound is from 2.5:1 to 1:2.5. Preferably the ratio is 1.5:1 to 1:1.5, and most preferably the ratio is 1:1.

In addition to the above components, the vehicle of the composition of the invention may contain small quantities of other excipients such as stabilizers or anti-oxidants to improve product stability, preservatives, artificial sweeteners, etc. Preferred anti-oxidants are disodium edetate (EDTA) and propyl gallate, preferably in quantities of 0.1% and 0.02% w/w, respectively. Preferred preservatives are potassium sorbate and alkyl parabens (methyl and propyl parahydroxyenzoates), wherein potassium sorbate may also help in increasing water solubility, if desired. The potassium sorbate is preferably used in the quantity of 0.2% w/w and the two paraben preservatives in the quantity of 0.2% and 0.05% w/w, respectively. The preferred sweetener is saccharin, preferably in the quantity of 0.1% w/w.

Advantageously, the vehicle composition may contain additional ingredients. One such example is a water-soluble synthetic polymer, preferably polyvinylpyrrolidone (or PVP, or povidone, or polyvidone), having suspending and/or viscosity-enhancement and/or film-forming activity. The polyvinylpyrrolidone is preferably used in a quantity of 8.000 to 10.000% (%w/w) and most preferably in the quantity of 9.000% (%w/w).

In another preferred embodiment in accordance with the present invention, the composition additionally includes a small quantity of at least one further biologically active compound, preferably selected from hyaluronic acid and derivatives thereof, including any pharmaceutically acceptable salt and in particular sodium hyaluronate; and 18-β-glycyrrhetinic acid. Hyaluronic acid, a naturally occurring mucopolysaccharide, and its sodium salt may be usefully employed in the composition of the invention due to their hydrating, lubricating and moisturizing properties, whereas 18-β-glycyrrhetinic acid (also known as Enoxolone) has anti-inflammatory and conditioning properties on the skin. The sodium hyaluronate is preferably used in the quantity of 0.01% and 18-β-glycyrrhetinic acid in the quantity of 0.03% by weight, on the total weight of the composition.

Polyvinylpyrrolidone, hyaluronic acid or its sodium salt, 18-β-glycyrrhetinic acid and their various possible combinations, together with the major ingredients of the water/glycol/macrogol-glycerol hydroxystearate vehicle identified above, can act synergistically to enhance the active ingredient availability at the affected area of the skin. In the formulation of the invention, clobetasol propionate penetrates the skin easily to diminish the psoriasis plaques and minimize inflammation.

A particularly preferred composition of the invention contains:

| **Ingredient:** | **Amount (%w/w)** |
|---|---|
| Clobetasol propionate | 0.025% |
| Water purified | 80.265% |
| Povidone K30 | 9.000% |
| Propylene glycol | 5.000% |
| Macrogolglycerol Hydroxystearate 40 | 5.000% |
| Potassium sorbate | 0.200% |
| Methyl parahydroxybenzoate | 0.200% |
| Propy parahydroxybenzoate | 0.050% |
| Disodium edentate | 0.100% |
| Saccharin sodium | 0.100% |
| Enoxolone | 0.030% |
| Propyl gallate | 0.020% |
| Sodium hyaluronate | 0.010% |
| | Tot. 100.000% |

The applicant has found that in the composition of the invention clobetasol propionate, diffused in the vehicle, performs a powerful anti-inflammatory effect and shows an optimal effectiveness in low concentration. In fact, the high efficiency of the formulation allows delivery of efficacious concentrations of the incorporated active ingredient to the skin and its persistence, so that it can better perform its therapeutic effects.

Accordingly, with respect to the prior art, the present invention provides a new, alternative formulation of clobetasol propionate at 0.025% w/w that is easy to prepare, by using conventional low cost ingredients, stable, easy to apply, and easily tolerated by patients to solve the compliance issues without compromising the required efficacy. Moreover, said formulation may permit more prolonged period of treatment without resulting in significant adverse effects.

As used herein, the terms "formulation" and "composition" are interchangeable.

As to a further discussion of the manner of usage and operation of the present invention, the same should be apparent from the above description. Accordingly, no further discussion relating to the manner of usage and operation will be provided.

With respect to the above description then, it is to be realized that the optimum dimensional relationships for the parts of the invention, to include variations in size, materials, shape, form, function and manner of operation, assembly and use, are deemed readily apparent and obvious to one skilled in the art.

Therefore, the foregoing is considered as illustrative only of the principles of the invention.

### Example 1

### Preparation of the clobetasol formulation

The composition of the product and the function of the materials are shown in Table 1.

**Table 1. The composition of the product and the function of the materials s**

| Maternal Name | Quantity [g/100g] | Function | Ref. to standard |
|---|---|---|---|
| Povidone K-30 | 9.00 | Binding agent | Ph Eur |
| Propylene glycol | 5.00 | Water miscible co-solvent | Ph Eur |
| Macrogolglycerol hydroxystearate (PEG-40 Hydrated Castor Oil) | 5.00 | Emilsifying agent | Ph Eur |
| Methyl parahydroxybenzoate | 0.20 | Antimicrobial preservatives | Ph Eur |
| Propyl parahydroxybenzoate | 0.0.5 | Antimicrobial preservative | It Eur |
| Potassium sorbate | 0.20 | Antimicrobial preservative | Pli Eur |
| Propyl gallat | 0.02 | Antioxidant | Ph Bur |
| Disodium edetate | 0.10 | Chelating agent | Ph Eur |
| Sodium hyaluronate | 0.01 | Moisturizer | Certificate of analysis |
| Saccharin sodium | 0.10 | Sweetening agent | Ph Eur |
| Enoxolone (Glycyrrhetinic acid) | 0.03 | Flavoring agent | Ph Eur |
| Clobetasol propionate | 0.025 | Active substance | BP |
| Water, Purified | Up to 100g | Solvent | Ph Eur |

The product is made in four phases, manufactured one by one, and finally mixed together.

### Phase I

The water was stirred and heated to 50 °C in a stainless steel vessel. Povidone K-30, saccarin sodium and disodium edetate were added and mixed until dissolved in the mentioned order. Each component was completely dissolved before next was added. The phase was cooled to 25° C and potassium sorbate was added.

### Phase II

In separate vessel, macrogolglycerol hydroxystearate was heated to 50°C. Clobetasol propionate is to be added while mixing to dissolve.

### Phase III

In a separate vessel propylene glycol was heated to 50°C. Methyl parahydroxybenzoate, propyl parahydroxybenzoate, propyl gallate and enoxolone (glycyrrhetinic acid) were added in the mentioned order while mixing to dissolve. Each component was completely dissolved before the next was added.

### Phase IV

In a separate vessel, sodium hyaluronate was dissolved in water at 25°C.

Phase II, III and IV were added to the Fryma Process Equipment (Fryma-Mashinen AG, Rheinfelden, Switzerland) in the mentioned order while mixing to dissolve.

The product was filled in 300 mL PET bottles with 300g in each bottle.

The product was stored at 5 C/amb, for not fewer than 12 h prior the filling. The product was filled in 300 ml PET bottles with 300 ml in each bottle. Not less than 30 bottles were filled.

The production, filling and labeling of the products took place at APLS, Apoteket AB, Production & Laboratories, Kungens kurva, Sweden.

### Example 2:

### Preparation of a stable formulation for also other active ingredients

This is the general process for producing a stable product for various active ingredients in an aqueous vehicle comprising propylene glycol as a solvent and moisture-retaining agent, and macrogol-glycerol hydroxystearate as a non-ionic emulsifier. The product is made in four phases, manufactured one by one, and finally mixed together.

### Phase I

The water was stirred and heated to 50 °C in a stainless steel vessel. Povidone K-30, saccarin sodium and disodium edetate were added and mixed until dissolved in the mentioned order. Each component was completely dissolved before next was added. The phase was cooled to 25° C and potassium sorbate was added.

### Phase II

In separate vessel, macrogolglycerol hydroxystearate was heated to 50°C. The active ingredient such as Clobetasol propionate in Example 1, is to be added while mixing to dissolve.

### Phase III

In a separate vessel propylene glycol was heated to 50°C. Methyl parahydroxybenzoate, propyl parahydroxybenzoate, propyl gallate and enoxolone (glycyrrhetinic acid) were added in the mentioned order while mixing to dissolve. Each component was completely dissolved before the next was added.

### Phase IV

In a separate vessel, sodium hyaluronate was dissolved in water at 25°C.

Phase II, III and IV were added to the Fryma Process Equipment in the mentioned order while mixing to dissolve.

The product was filled in 300 mL PET bottles with 300g in each bottle.

The product was stored at 5 C/amb, for not fewer than 12 h prior the filling. The product was filled in 300 ml PET bottles with 300 ml in each bottle. Not less than 30 bottles were filled.

The production, filling and labeling of the products took place at APLS, Apoteket AB, Production & Laboratories, Kungens kurva, Sweden.

### Example 3

### Stability test of clobetasol formulation

The aim of this test was to establish the shelf life of clobetasol propionate 0.025 % oromucosal gel. Physical, chemical and microbiological tests on the product were performed in order to verify and document the stability. The study was performed on a technical batch manufactured in a Fryma Process Equipment pilot-plant.

The product was manufactured according to EXAMPLE 1.

The product was filled in 300 ml PET bottles (PET Power art. no. 02803001A) with tamper-proof cap (PET Power art. no. 201539-2PE). Samples were stored for 24 months at 5 °C/ amb and 25°C/60% RH respectively and for 6 months at 40C°/75% RH for up to 6 months.

The product has been subjected to the following tests: appearance (microscope and visual), pH, total viable aerobic count, absence of *E. coli,* viscosity and assay of clobetasol propionate, methyl parahydroxybenzoate, propyl parahydroxybenzoate and potassium sorbate.

### Result

The amount of active substance (cobetasol propionate) is within limits (0.0225-0.0275 % (w/w)) at all examinations. The amount varied between 0.0241-0.0250% (w/w). The amount of the preservatives (methyl parahydroxybenzoate, propyl parahydroxybenzoate and potassium sorbate) was within limits at all examinations. No significant changes in content of preservatives were observed. No degradation products were found.

The visual examinations show a temperature dependent change in appearance. Initially, the product consists of slightly yellow transparent liquid. After six months of storage at 25°C/60% RH and two months of storage at 40C°/75% RH, a clearer yellow color was observed. The change in color is more noticeable at 40C°/75% RH than at 25°C/60% RH .

However, the product is homogeneous and transparent at all examinations. No significant change in appearance has been noted between six and 24 months of storage at 25 °C/60% RH and between two and six months of storage at 40C°/75% RH respectively. No significant change in appearance has been noted at 5/amb. The origin of the yellow color derivates from Povidone K30 dissolved in water. The change in appearance is expected to be acceptable for the patient. No significant changes in appearance in the microscope have been observed and all examinations were within limits.

The pH was within limits (4.5-7.0) and varied between 5.3-5.5 at all examinations.

The microbiological examinations were within limits at all examinations. No *Escherichia coli* was found initially or after storage. Less than 1 cfu/g bacteria and fungi were found at all examinations.

No change in viscosity dependent on storage temperature or storage time has been observed.

The results obtained from this stability study indicate a shelf-life of clobetasol propionate 0.025 % oromucosal gel of at least 24 months at 25°C/60% RH.

### Example 4

### Use of clobetasol formulation for treatment of psoriasis

The clobetasol formulation is used topically in the form of a spray for treating psoriasis. The formulation is sprayed on the affected areas, avoiding healthy skin, one time per day until satisfactory results are achieved. Following this initial regime, a maintenance dose, once a week, is used on the affected skin areas.

### Example 5

### Use of clobetasol formulation for treatment of lichen planus

The clobetasol formulation is used orally to treat lichen planus in a six-week treatment regime. During the first two weeks, 5 ml of the clobetasol formulation is gargled morning and evening. The following two weeks, 5 ml of the formulation is gargled once per day. During weeks five and six, 5 ml of the clobetasol formulation is gargled once every two days until the end of the treatment regime.

## Claims

1. A pharmaceutical composition for topical use, comprising 0.005% to 0.05 % by weight of 17-clobetasol propionate, in an aqueous vehicle comprising propylene glycol as a solvent and moisture-retaining agent, and an emulsifier, wherein the emulsifier consists of macrogol-glycerol hydroxystearate.

2. The pharmaceutical composition according to claim 1, comprising 0.02 to 0.025 % by weight of 17-clobetasol propionate.

3. The pharmaceutical composition according to claims 1, comprising 0.025% by weight of 17-clobetasol propionate.

4. The pharmaceutical composition according to claim 1, wherein the propylene glycol is present in a proportion between 4% and 10%, water in a proportion between 70% and 85% and macrogol-glycerol hydroxystearate in a proportion between 4% and 10%.

5. The pharmaceutical composition according to claim 1, wherein the propylene glycol is present in the amount of 5.000 % (%w/w), water in the amount of 80% and macrogol-glycerol hydroxystearate in the amount of 5.000 % (%w/w).

6. The pharmaceutical composition according to claim 4, further containing disodium edetate and/or propyl gallate as anti-oxidants.

7. The pharmaceutical composition according to claim 6, wherein disodium edetate and/or propyl gallate are present in quantities of 0.1% w/w and 0.02% w/w, respectively.

8. The pharmaceutical composition according to claim 1 further containing a preservative, selected from the group consisting of potassium sorbate and alkyl parahydroxybenzoates and/or a sweetener.

9. The pharmaceutical composition according to claim 8, containing potassium sorbate in the quantity of 0.2% w/w.

10. The pharmaceutical composition according to claim 4, containing a further ingredient selected from hyaluronic acid, a pharmaceutically acceptable salt of hyaluronic acid, and 18-β-glycyrrhetinic acid.

11. The pharmaceutical composition according to claim 4, in the form of lotion, gel, oromucosal gel, cream, ointment, oil, emulsion, solution, liquid suspension or dispersion, or spray.

12. A pharmaceutical composition according to any one of claims 1 to 11 for use in the treatment of skin and mucous membrane conditions associated with disorders including lichen planus, psoriasis, eczema, contact dermatitis, atopic dermatitis; seborrheic dermatitis, and other forms of dermatitis.

## Patentansprüche

1. Pharmazeutische Zusammensetzung zur topischen Verwendung, die 0,005 bis 0,05 Gew.-% 17-Clobetasolpropionat in einem wässrigen Träger, der Propylenglykol als Lösungsmittel und ein Feuchthaltemittel umfasst, und einen Emulgator umfasst, wobei der Emulgator aus Macrogol-Glycerinhydroxystearat besteht.

2. Pharmazeutische Zusammensetzung nach Anspruch 1, die 0,02 bis 0,025 Gew.-% 17-Clobetasolpropionat umfasst.

3. Pharmazeutische Zusammensetzung nach Anspruch 1, die 0,025 Gew.-% 17-Clobetasolpropionat umfasst.

4. Pharmazeutische Zusammensetzung nach Anspruch 1, bei der das Propylenglykol in einem Anteil zwischen 4 % und 10% vorliegt, Wasser in einem Anteil zwischen 70 % und 85 % vorliegt und Macrogol-Glycerinhydroxystearat in einem Anteil zwischen 4 % und 10% vorliegt.

5. Pharmazeutische Zusammensetzung nach Anspruch 1, bei der das Propylenglykol in einer Menge von 5,000 % (% Gew./Gew.) vorliegt, Wasser in einer Menge von 80 % vorliegt und Macrogol-Glycerinhydroxystearat in einer Menge von 5,000 % (% Gew./Gew.) vorliegt.

6. Pharmazeutische Zusammensetzung nach Anspruch 4, die ferner Dinatriumedetat und/oder Propylgallat als Antioxidationsmittel enthält.

7. Pharmazeutische Zusammensetzung nach Anspruch 6, bei der Dinatriumedetat und/oder Propylgallat in Mengen von 0,1% Gew./Gew. bzw. 0,02 % Gew./Gew. vorliegen.

8. Pharmazeutische Zusammensetzung nach Anspruch 1, die ferner ein Konservierungsmittel, das aus der Gruppe, bestehend aus Kaliumsorbat und Alkylparahydroxybenzoaten, ausgewählt ist, und/oder ein Süßungsmittel enthält.

9. Pharmazeutische Zusammensetzung nach Anspruch 8, die Kaliumsorbat in der Menge von 0,2 % Gew./Gew. enthält.

10. Pharmazeutische Zusammensetzung nach Anspruch 4, die einen weiteren Bestandteil enthält, der aus Hyaluronsäure, einem pharmazeutisch verträglichen Salz von Hyaluronsäure und 18-β-Glycyrrhetinsäure ausgewählt ist.

11. Pharmazeutische Zusammensetzung nach Anspruch 4 in der Form einer Lotion, eines Gels, eines Mundschleimhautgels, einer Creme, einer Salbe, eines Öls, einer Emulsion, einer Lösung, einer flüssigen Suspension oder Dispersion oder eines Sprays.

12. Pharmazeutische Zusammensetzung nach einem der Ansprüche 1 bis 11 zur Verwendung bei der Behandlung von Haut- und Schleimhautmembranzuständen, die mit Erkrankungen zusammenhängen, die Lichen planus, Psoriasis, Ekzem, Kontaktdermatitis, atopische Dermatitis, Seborrhoe und andere Formen von Dermatitis umfassen.

## Revendications

1. Composition pharmaceutique pour une utilisation topique, comprenant 0,005 % à 0,05 % en poids de propionate de 17-clobétasol, dans un véhicule aqueux comprenant du propylène glycol comme solvant et agent conservant l'humidité, et un émulsifiant, l'émulsifiant consistant en hydroxystéarate de macrogol-glycérol.

2. Composition pharmaceutique selon la revendication 1, comprenant 0,02 à 0,025 % en poids de propionate de 17-clobétasol.

3. Composition pharmaceutique selon la revendication 1, comprenant 0,025 % en poids de propionate de 17-clobétasol.

4. Composition pharmaceutique selon la revendication 1, dans lequel le propylène glycol est présent dans une proportion entre 4 % et 10 %, l'eau, dans une proportion entre 70 % et 85 %, et l'hydroxystéarate de macrogol-glycérol, dans une proportion entre 4 % et 10 %.

5. Composition pharmaceutique selon la revendication 1, dans lequel le propylène glycol est présent dans la quantité de 5,000 % (% p/p), l'eau, dans la quantité de 80 % et l'hydroxystéarate de macrogol-glycérol, dans la quantité de 5,000 % (% p/p).

6. Composition pharmaceutique selon la revendication 4, comprenant en outre de l'édétate disodique et/ou du gallate de propyle comme anti-oxydants.

7. Composition pharmaceutique selon la revendication 6, dans laquelle l'édétate disodique et/ou le gallate de propyle sont présents dans des quantités respectivement de 0,1 % p/p et 0,02 % p/p,.

8. Composition pharmaceutique selon la revendication 1, comprenant en outre un conservateur, choisi dans le groupe consistant en sorbate de potassium et parahydroxybenzoates d'alkyle et/ou un agent édulcorant.

9. Composition pharmaceutique selon la revendication 8, contenant du sorbate de potassium dans la quantité de 0,2 % p/p.

10. Composition pharmaceutique selon la revendication 4, contenant un autre ingrédient choisi parmi l'acide hyaluronique, un sel pharmaceutiquement acceptable de l'acide hyaluronique, et l'acide 18-β-glycyrrhétinique.

11. Composition pharmaceutique selon la revendication 4, sous la forme d'une lotion, d'un gel, d'un gel oromucosal, d'une crème, d'une pommade, d'une huile, d'une émulsion, d'une solution, d'une suspension ou dispersion liquide, ou d'une pulvérisation.

12. Composition pharmaceutique selon l'une quelconque des revendications 1 à 11 pour une utilisation dans le traitement d'états de la peau et des membranes muqueuses associés aux troubles comprenant le lichen plan, le psoriasis, l'eczéma, la dermatite de contact, la dermatite atopique ; la dermatite séborrhéique ; et autres formes de dermatite.
